(19) Europäisches Patentamt
European Patent Office
Office européen des brevets

(11) EP 2 985 043 A1

(12) EUROPEAN PATENT APPLICATION

(43) Date of publication:
**17.02.2016 Bulletin 2016/07**

(21) Application number: **14382315.1**

(22) Date of filing: **14.08.2014**

(51) Int Cl.:
***A61L 31/10*** *(2006.01)* ***A61L 31/16*** *(2006.01)*
***C23C 18/16*** *(2006.01)* ***C23C 18/18*** *(2006.01)*
***C23C 18/20*** *(2006.01)* ***B05D 1/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Institut d'Investigació Biomèdica de Bellvitge (IDIBELL)**
  **08908 Hospitalet de Llobregat (ES)**
- **Institut Químic de Sarriá CETS Fundació Privada**
  **08017 Barcelona (ES)**

(72) Inventors:
- **Rosell Gratacos, Antoni**
  **08908 Hospitalet de Llobregat (ES)**
- **Borros Gómez, Salvador**
  **08017 Barcelona (ES)**
- **Gilabert Porres, Joan**
  **08017 Barcelona (ES)**
- **Martí Martí, Sara**
  **08908 Hospitalet de Llobregat (ES)**
- **Montes Worboys, Ana**
  **08908 Hospitalet de Llobregat (ES)**
- **Ramos Pérez, Víctor**
  **08017 Barcelona (ES)**
- **Molina Molina, María**
  **08908 Hospitalet de Llobregat (ES)**

(74) Representative: **ABG Patentes, S.L.**
**Avenida de Burgos, 16D**
**Edificio Euromor**
**28036 Madrid (ES)**

# (54) Process for manufacturing a customizable medical device and device obtained by said process

(57) The invention relates to medical devices and, more in particular, to medical devices suitable for use as stents that contain an antimicrobial coating and, optionally, a drug customized to patients requirements. The invention also relates to methods for obtaining devices having the above features.

EP 2 985 043 A1

## Description

### FIELD OF THE INVENTION

The invention relates to medical devices and, more in particular, to medical devices suitable for use as stents that contain an antimicrobial coating and, optionally, a drug customized to patients requirements. The invention also relates to methods for obtaining devices having the above features.

### BACKGROUND OF THE INVENTION

Different benign and malignant conditions can cause tracheobronchial stenosis which result in impaired breathing with dyspnea and if not treated, asphyxia and death. Tracheobronchial stents are designed to mechanically stabilize the lumen once normalized by dilatation or debulking.

Post inflammatory tracheal stenosis is the most common non-malignant tracheal injury and is usually caused by prolonged ischemia and usually concomitant infection that cause necrosis of the tracheal wall. Repair of the damaged wall can result in abnormal formation of granulation tissue and stenotic scarring. This situation requires restoration of the lumen by complex surgery of the trachea or endoscopic dilatation with or without tissue ablation or insertion of silicon trachea prosthesis (stent). Current endoscopic treatment has a high rate of recurrence.

Malignant tracheal stenosis can be originated by primary tumors of the chest (lung, pleurae, esophagus) or by metastasis. Different debulking methods can be endoscopically used to recover lumen patency, and once reopened, stents are implanted to avoid restenosis. The only propriety of current stents is mechanical compression.

One of the most common complications of stent implantation is bacterial colonization by potentially pathogenic microorganisms (PPM), like *Pseudomonas aeruginosa* or *Staphylococcus aureus.* Bacterial colonization can reduce quality of life (i.e. halitosis, productive cough...); induce infection or even sepsis that can cause death of the patient, if appropriate care is not taken.

Bacterial colonization and biofilm formation of the implanted medical devices are a serious problem accounting for over 80% of intrahospital microbial infections. Although antibiotics can treat the great majority of them, their continuous use can generate resistance, increase the cost and produce adverse effects on patients. Prevention by avoiding biofilm formation by means of covering catheters and tubs with different antimicrobials like silver can be more convenient. Silver has a high toxicity against microorganisms, wide spectra of action, non-bacterial resistance and it is not toxic to animal cells.

Another complication is granuloma formation at the edges of the stents consequence of mechanical stress. This inflammatory tissue can be a life threatening complication if not treated with glucocorticoids or endoscopic thermal ablation. Therefore, delivering antiproliferative drug at both edges of the stent could reduce or avoid this complication (Choong CK et al, J Thorac Cardiovasc Surg. 2006, 131(1):60- 4).

Recurrence of tracheobronchial stenosis after stent removal is seen in nearly half of the patients (Maldonado F, et al, Laryngoscope. 2014, 124(2):498-503). To avoid this, some topical antiproliferative agents like mitomice have been used with apparent good respond, mainly in children (Ortiz R, et al., Eur J Pediatr Surg. 2014, 24(1):39-45). It's seems reasonable to expect that a prolonged topical treatment with an antiproliferative drug, like placlitaxel, coating the outside of the stent could reduce the time of implantation and even completely heal the stricture (Zhu GH, et al, Laryngoscope. 2011,121(10):2234-9).

Antiproliferative coating drugs like cisplatin, have also been proposed for cancer patients, after successful animal model assays (Chao YK, et al, Chest. 2013, 144(1):193-9). These initial experiments open the possibility to cover stents with personalized pharmacological treatments according to the type or mutation status of the cancer.

There is an increasing need of discover and design new materials capable to interact with the body tissue and presenting customizable surfaces which can be provided with different biological activities.

### SUMMARY OF THE INVENTION

The authors of the present invention have developed antibacterial PDMS surfaces for sustained silver ions release creating an antibacterial coating and avoiding biofilm friendly surfaces that has potential applicability on implanted medical devices. PDMS are coated with PFM through PECVD technique and incubated with amine sugar (glucosamine). As a consequence of the sugar aldehyde, silver ions are reduced on the surface to obtain a silver coated surface. The silver coating proposed is able to adapt to a flexible surface (like medical silicone) keeping its adherence to the polymeric surface even after the polymeric device is strongly twisted. At the same time, the device can be pharmacologically customized covering the chosen surface with the required drug. In a more specific manner, the drug can be attached to the stent surface in an encapsulated form to obtain a controlled release to treat scarring tissue or granuloma formation, at the desired location. The method described in the present invention can be applied for the pharmacological activation

of any stent which is intended for its use in any organ or hollow tissue, including not only the respiratory tract, but also the digestive tract, the biliary tract, the urinary tract or the vascular system.

LEGENDS TO FIGURES

**Figure 1.** ToF-SIMS of PDMS samples with different coatings. Non-modified PDMS samples (PDMS), PDMS coated with PFM (PFM), PDMS coated with PFM and incubated with glucosamine (Glucosamine), PDMS coated with PFM and incubated with milliQ water (Water) and PDMS coated with PFM, incubated with glucosamine and silver deposition (Silver). (A) ToF-SIMS spectra of positive ions (normalized by m/z = 28) and (B) ToF-SIMS spectra of negative ions (normalized by m/z = 60).
**Figure 2.** Silver release of PDMS samples in TSB media and milliQ in oxic conditions.
**Figure 3.** Bacterial activity of non-coated (PDMS) and coated (silver) samples. (A) Cell viability comparison between different bacteria strains. Results were obtained analyzing previous images using imageJ software. (B) Viability of different supernatant strains after being incubated with silver samples.
**Figure 4.** Cell viability of different cell lines type with paclitaxel loaded nanoparticles. (A) Human lung normal fibroblast (IMR-90) incubated with Ctrl (control cells), Ø (free drug nanoparticles), 1% and 3% paclitaxel content nanoparticles and PCTXL (pure paclitaxel) at 14 nM. (B) Stenotic fibroblast incubated with Ctrl (control cells), Ø (free drug nanoparticles), 1% and 3% paclitaxel containing nanoparticles and PCTXL (pure paclitaxel) at 14 nM.

DETAILED DESCRIPTION OF THE INVENTION

*Method for coating a substrate with a metallic layer*

In a first aspect, the invention relates to a method for coating a substrate with a metallic ion, hereinafter second method of the invention, comprising the steps of

(i) Contacting at least a first surface of the substrate with a reactive monomer containing an activated carboxyl group under conditions adequate for the formation of a polymer coat containing said activated carboxyl group on the surface of the substrate by polymerization of the monomer,
(ii) Contacting the substrate obtained in step (i) with a reducing carbohydrate comprising a group reactive with said activated carboxyl group under conditions adequate for the formation of a covalent bond between the reactive group in the carbohydrate and the activated carboxyl group on the surface of the substrate, thereby obtaining a surface modified with a reducing carbohydrate and
(iii) Contacting the surface obtained in step (ii) with a salt of said metallic ion under conditions adequate for the reduction of the metallic ions by the reducing carbohydrate and the deposition of the metallic ions on the surface, thereby obtaining a surface coated with the metallic ion.

The term "coating", as used herein, refers to a layer that is either naturally or synthetically formed on or applied to the surface of the substrate.
The term "substrate", as used herein, refers to any material whose surface can be coated.
In a particular embodiment, the substrate is a polymeric substrate.
The term "polymeric substrate", as used herein, refers to a substrate of a chemical compound or mixture of compounds consisting of repeating structural units created only through a process of polymerization with a molecular weight higher than 6.000 which is solid at ambient temperature. Polymers that contain only a single type of repeat unit are known as homopolymers, while polymers containing a mixture of repeat units are known as heteropolymers or copolymers. In a more particular embodiment, the polymeric substrate is a silicone-based polymer.
The term "silicone-based polymer", as used herein, refers to a polymer material comprising at least two organosiloxane units independently selected from ($R_3SiO_{1/2}$), ($R_2SiO_{2/2}$), ($RSiO_{1.5}$), or ($SiO_2$) siloxy units (commonly known as M, D, T, and Q siloxy units respectively), where R typically represents a saturated alkyl group containing 1-6 carbon atoms such as methyl, or an aryl group containing 6-10 carbon atoms such as phenyl. The polymer may comprise additional groups that may be chosen from the following groups: -COOH, - $COO^-$, -COO-, -OH, -$NH_2$, -NH-, -NR-, -$SO_3H$, -$SO_3^-$,-$OCH_2CH_2$-, -O-$CH_2CH_2CH_2$-, -O-$CH_2CH(CH_3)$-, -$NR_3^+$, -SH, -$NO_2$, -I, -Cl, Br, -CN, - $PO_4^{3-}$, -CONH-, -CONR-, -$CONH_2$, - CSNH-, -$SO_2$-, -SO-, -$SO_2NH$-, -NHCO-,-$NHSO_2$-, -NHCOO-, -OCONH-, -NHCSO- and -OCSNH-, R representing an alkyl group. Examples of silicone-based polymers corresponding to the definition are especially polydimethylslloxanes (PDMS).
Preferably, the number-average molecular mass of the silicone polymers containing a polysiloxane backbone

ranges from 10.000 to 1.000.000 approximately and even more preferentially from 10.000 to 100.000 approximately.

In another particular embodiment, the substrate is a metallic substrate.

In another particular embodiment, the substrate is a metallic substrate coated with a polymer substrate, preferably a silicone-based polymer substrate.

In a first step of the first method of the invention, the substrate is contacted with a reactive monomer containing an activated carboxyl group under conditions adequate for the coating of the surface of the substrate with said molecule. This step is known as the monomer deposition step and consists in the reaction of the monomer with the activated surface and its polymerization to form a coating. As described herein, a monomer containing an activated carboxyl group is deposited on the target silicone-based polymer substrate to form a coating.

Reactive monomers containing an activated carboxyl group for use according to the present invention include, without limitation, acrylic acid monomers and methacrylic acid monomers.

The term "reactive monomer carrying a reactive carboxylic acid group" refers to molecules having the general structure:

R-CO-L

wherein R is a group capable of cationic, anionic or free radical polymerization and L is a leaving group.

These molecules can be cross-linked through the R groups resulting in a polymer characterized in that it contains in each monomer unit at least one activated carboxyl acid group.

Suitable R groups include monovalent groups that can undergo free radical and/or cationic polymerization comprising up to 20 carbon atoms. Preferred R groups comprise free radical reactive groups, such as acrylates, styryls, vinyls, vinyl ethers, $C_{1-6}$alkylacrylates, acrylamides, $C_{1-6}$alkylacrylamides, N-vinyllactams, N-vinylamides, $C_{2-12}$alkenyls, $C_{2-12}$alkenylphenyls, $C_{2-12}$alkenylnaphthyls, or $C_{2-6}$alkenylphenyl, $C_{1-6}$alkyls or a cationic reactive group such as vinyl ethers or epoxide groups and mixtures thereof. In a preferred embodiment, the R group is a methacrylate.

Suitable L groups are stable under reaction conditions, and protect the carboxylate group and leave readily when contacted with a reactive group (e.g., an amine group). Suitable L groups include alkyl esters, phenyl esters, hydroxy para-nitroaryls p-nitrophenyl esters, N-hydroxylamine derivatives, and tosylate esters all of which may be substituted or unsubstituted. Preferred L groups include t-butyl esters, 2,4,5-trichlorophenyl esters, pentafluorophenyl esters, N-hydroxysuccinimide esters, N-hydroxy-oxo-dihydrobenzotriazine derivatives, 1-hydroxybenzotriazole esters, tosylate esters, and combinations thereof. Preferred suitable L groups include pentafluorophenyl esters, tosylate esters, and N-hydroxysuccinimide esters, and mixtures thereof. Preferred latent reactive compounds include pentafluoromethacrylate and N-acryloxysuccinimide and mixtures thereof and the like.

In a preferred embodiment, the R group is a methacrylate. In another preferred embodiment, the L group is a pentafluorophenyl. In a still more preferred embodiment, the reactive monomer containing an activated carboxyl group is pentafluorophenyl methacrylate (PFM).

In a preferred embodiment, the monomer deposition process is carried out by vapor deposition process.

As used herein, the terms "deposition process" and "vapor deposition process" refer to a process in which a polymeric layer is formed on one or more surfaces of a substrate from vaporized precursor composition(s) including one or more monomers. The monomers are vaporized and directed to and/or contacted with one or more surfaces of a substrate (e.g., semiconductor substrate or substrate assembly) placed in a deposition chamber. In a particular embodiment, the substrate is heated. These monomer compounds form a non-volatile, thin, uniform, layer on the surface(s) of the substrate. As used herein, a "vapor deposition process" can be a chemical vapor deposition process (including pulsed chemical vapor deposition processes) or an atomic layer deposition process.

In a preferred embodiment the vapor deposition process is a chemical vapor deposition.

As used herein, the term "chemical vapor deposition" or "CVD" refers to a vapor deposition process wherein the desired layer is deposited on the substrate from vaporized metal-containing compounds (and any reaction gases used) within a deposition chamber with no effort made to separate the reaction components. CVD is a process whereby atoms or molecules are deposited in association with a chemical reaction (e.g., a reduction reaction, an oxidation reaction, a decomposition reaction, etc.) of vapor-phase precursor species. When the pressure is less than atmospheric pressure, CVD processes are sometimes referred to as low-pressure chemical vapor deposition (LPCVD) processes. Plasma-enhanced chemical vapor deposition (PECVD) techniques are chemical vapor deposition techniques in which plasma is employed such that the precursor gas is at least partially ionized, thereby typically reducing the temperature that is required for chemical reaction. Chemical vapor deposition processes are not necessarily line-of-site processes, allowing coatings to be formed on substrates of complex geometry. PEVCD can be carried out according to any technique known in the art, such as the methods described by Duque et al., Plasma Prowess. Polym. 2010, 7, 915-925), Francesch; E. et al., (Plasma Process. Polym. 2005, 2, 605-611), Langer; D. and Tirrell (Nature 2004, 428, 487-492) or Duque et al. Biomacromolecules 2010, 11, 2818-2823).

The flow rate of the monomer can be varied in the deposition method. In certain embodiments, the flow rate of

the first monomer is about 10 sccm, about 20 sccm, from about 30 sccm to about 0.01 sccm, from about 20 sccm to about 0.05 sccm, from about 10 sccm to about 0.1 sccm, about 30 sccm, about 28 sccm, about 26 sccm, about 24 sccm, about 22 sccm, about 20 sccm, about 18 sccm, about 16 sccm, about 14 sccm, about 12 sccm, or about 10 sccm, about 9 sccm, about 8 sccm, about 7 sccm, about 6 sccm, about 5 sccm, about 4 sccm, about 3 sccm, about 2 sccm, or about 1 sccm. In certain embodiments, the flow rate of the reactive monomer is about 0.9 sccm, about 0.8 sccm, about 0.7 sccm, about 0.6 sccm, about 0.5 sccm, about 0.4 sccm, about 0.3 sccm, about 0.2 sccm, about 0.1 sccm, about 0.05 sccm, or about 0.01 sccm.

In certain embodiments, the rate of polymer deposition is about 1 micron/minute, between about 1 micron/minute and about 100 nm/minute, between about 10 micron/minute and about 100 nm/minute or between about 100 micron/minute and about 100 nm/minute.

The coating process can take place at a range of temperatures. In certain embodiments, the temperature of the substrate is ambient temperature. In certain embodiments, the temperature is about -20°C, about -10 °C, about 0°C, about 10°C, about 20 °C, about 30°C, about 40 °C, about 50 °C, about 60 °C, about 70 °C, about 80 °C, about 90 °C, about 100 °C, about 110 °C.

When the coating process is carried out by PECVD, the radiofrequency is applied at a power of at least 1 W, at least 2 W, at least 3 W, at least 4 W, at least 5 W, at least 6W, at least 7W, at least 8W, at least 9W, at least 10W, at least 15W, at least 15 W, at least 20 W, at least 25 W, at least 30 W, at least 35W, at least 40 W, at least 45 W, at least 50W or at least 55W, at least 60 W, at least 65W, at least 70W more, at least 75 W, at least 80W, at least 85 W, at least 90 W, at least 95 W, at least 100 W or more.

Alternatively, the coating process by PECVD can be performed by using other source of excitation instead of radiofrequency, such as microwaves or direct current.

The plasma may operate at low, sub-atmospheric or atmospheric pressures as are known in the art. The monomer may be introduced into the plasma as a vapor or an atomized spray of liquid droplets (WO03101621 and WO03097245, Surface Innovations Limited). The monomer may be introduced into the pulsed plasma deposition apparatus continuously or in a pulsed manner by way of, for example, a gas pulsing valve

The substrate to which the coating is applied will preferentially be located substantially inside the pulsed plasma during coating deposition. However, the substrate may alternatively be located outside of the pulsed plasma, thus avoiding excessive damage to the substrate or growing coating.

The monomer will typically be directly excited within the plasma discharge. However, "remote" plasma deposition methods may be used as are known in the art. In said methods the monomer enters the deposition apparatus substantially "downstream" of the pulsed plasma, thus reducing the potentially harmful effects of bombardment by short-lived, high-energy species such as ions.

The plasma may comprise the monomeric compound alone, in the absence of other compounds or in admixture with for example an inert gas. Plasmas consisting of monomeric compound alone may be achieved as illustrated hereinafter, by first evacuating the reactor vessel as far as possible, and then purging the reactor vessel with the organic compound for a period sufficient to ensure that the vessel is substantially free of other gases. The temperature in the plasma chamber is suitably high enough to allow sufficient monomer in gaseous phase to enter the plasma chamber. This will depend upon the monomer and conveniently ambient temperature will be employed. However, elevated temperatures for example from 25 to 250 degrees centigrade may be required in some cases.

In alternative embodiments of the invention, materials additional to the plasma polymer coating precursor are present within the plasma deposition apparatus. The additional materials may be introduced into the coating deposition apparatus continuously or in a pulsed manner by way of, for example, a gas pulsing valve.

The additive materials may be inert and act as buffers without any of their atomic structure being incorporated into the growing plasma polymer (suitable examples include the noble gases). A buffer of this type may be necessary to maintain a required process pressure. Alternatively the inert buffer may be required to sustain the plasma discharge. For example, the operation of atmospheric pressure glow discharge (APGD) plasmas often requires large quantities of helium. This helium diluent maintains the plasma by means of a Penning Ionization mechanism without becoming incorporated within the deposited coating.

In other embodiments of the invention, the additive materials possess the capability to modify and/or be incorporated into the coating forming material and/or the resultant plasma deposited coating. Suitable examples include other reactive gases such as halogens, oxygen, and ammonia.

In a <u>second step of the first method of the invention,</u> the method of the invention comprises contacting the substrate obtained in step (i) with a reducing carbohydrate comprising a group reactive with the activated carboxyl group under conditions adequate for the formation of a covalent bond between the reactive group in the carbohydrate and the carboxyl group on the monomeric units of the polymer, with the release of the L group, thereby obtaining an surface modified with a reducing monosaccharide.

The term <u>"carbohydrate"</u>, also known as "saccharide" or "sugar", refers to a macromolecule of carbon, hydrogen and oxygen and includes monosaccharides, disaccharides, oligosaccharides, and polysaccharides. The term "monosaccharide", as used herein, refers to a simple form of a sugar that consists of a single saccharide unit which cannot be further decomposed to smaller saccharide building blocks or moieties, including without limitation furanose, fructose, glucose, galactose, mannose, a modified monosaccharide, sialic acid and eritrose and mixtures thereof.

The term "reducing carbohydrate comprising a group reactive with a carbonyl group", as used herein, refers to any carbohydrate which (i) at least comprises one group reactive with a carboxyl group and (ii) the aldehyde or a ketone is in its open chain form. This allows the carbohydrate to act as a reducing agent, for example in a reaction with an amine. A carbohydrate may be a reducing sugar when its anomeric carbon (the carbon linked to two oxygen atoms) is in the free form. Carbohydrates may occur in a chain as well as a ring structure and it is possible to have equilibrium between these two forms. Further, some keto carbohydrates are reducing carbohydrates because they may be converted to an aldehyde via a series of tautomeric shifts to migrate the carbonyl to the end of the chain. This pathway could become accessible during the thermal curing process. Examples of reducing carbohydrates include monosaccharides, whether aldose (containing an aldehyde) or ketose (containing a ketone), including glucose, fructose, glyceraldehydes, lactose, arabinose and maltose. Accordingly, the reducing carbohydrate component of the present invention may be a monosaccharide in its aldose or ketose form, including a triose, a tetrose, a pentose, a hexose, or a heptose. Glyceraldehyde and dihydroxyacetone are considered to be aldose and ketose sugars, respectively. Examples of aldotetrose sugars include erythrose and threose; and ketotetrose sugars include erythrulose. Aldopentose sugars include ribose, arabinose, xylose, and lyxose; and ketopentose sugars include ribulose, arabulose, xylulose, and lyxulose. Examples of aldohexose sugars include glucose (for example, dextrose), mannose, galactose, allose, altrose, talose, gulose, and idose; and ketohexose sugars include fructose, psicose, sorbose, and tagatose. Ketoheptose sugars include sedoheptulose. In one embodiment, the reducing carbohydrate comprising a group reactive with the carboxyl group is a non-modified carbohydrate having the general formula $Cm(H2O)n$ wherein m and n are the same of different and wherein the group reactive with the carboxyl group is one of the groups which occurs naturally in the carbohydarte (e.g. the hydroxyl groups). In another embodiment, the reducing carbohydrate comprising a group reactive with the carboxyl group is a modified carbohydrate wherein the group which reacts with the carboxyl group appears in the modification. In a more preferred embodiment, the modified carbohydrate is N-glucosamine, being the group which reacts with the carboxyl group the amine group.

In another particular embodiment, the reducing carbohydrate is a reducing disaccharide. Most disaccharides are also reducing sugars. Other natural or synthetic stereoisomers or optical isomers of reducing sugars may also be useful as the reducing sugar component of the aqueous binder composition; for example, dextrose, which is one of the optical isomers of glucose. In addition, the reducing sugar can comprise a dehydrated form of a reducing mono- or disaccharide, such as, hydroxymethyl furfural from dehyrdrogenated glucose. Further, a number of suitable reducing sugar sources may be used, such as corn syrup, high fructose corn syrup, and other fructose and dextrose equivalents.

The reducing monosaccharide may also be substituted, for example, with hydroxy, halo, alkyl, alkoxy, carbonyl or other substituent groups.

The reducing carbohydrate of the invention is modified with a group reactive with an activated carboxyl group.

The term "group reactive with an activated carboxyl group", as used herein, refers to a group that is capable of reacting with an activated carboxyl group to form a covalent bond, i.e. is covalently reactive under suitable reaction conditions, and generally represents a point of attachment for another substance. Exemplary reactive groups include, but not limited to amines, hydroxyl groups, thiols, carboxylic acids and esters.

The group reactive with an activated carboxyl group can be either one of the hydroxyl groups of the reducing carbohydrate or a group which has been linked to the carbohydrate by chemical modification, in which case the reducing carbohydrate will be a reducing carbohydrate modified with a group reactive with an activated carboxyl group.

In a particular embodiment, the reactive group is an amino group and the covalent bond between said amino group in the carbohydrate and the activated carboxyl group on the surface of the substrate is an amide bond.

Reducing monosaccharides modified with an amino group suitable for use according to the present invention include, without limitation, glucosamine, N-acetyl glucosamine, mannosamine, N-acetyl mannosamine, galactosamine, N-acetyl galactosamine, their isomers (e.g., stereoisomers), and their salts (e.g., HCl salt). In one embodiment, the sugar amine is glucosamine, or D-glucosamine and N-acetyl glucosamine, or N-acetyl-D-glucosamine. Additionally, combinations of two or more sugar amines may be used.

The term "glucosamine", as used herein, refers to the compound β-D-glucosamine.

The contacting step between the substrate obtained in step (i) of the present invention and the reducing monosaccharide modified with an amino group is carried out for sufficient time so as to allow the modification of at least 1%, at least 10%, at least 20%, at least 30%, at least 40%, at least 50%, at least 60%, at least 70%, at least 80%, at least 90%, at least 95% or 100% of the reactive carboxyl groups on the polymeric surface coating obtained in step (i). Accordingly, the reaction with the reducing monosaccharide modified with an amino group is carried out for at least 1 h, at least 2 h, at least 3 h, at least 4 h, at least 5 h, at least 6h, at least 7h, at least 8h, at least 8h, at least 10 or more.

In a third step of the method of the invention, the substrate obtained in step (ii) is contacted with a salt of said metallic ion under conditions adequate for the reduction of the metallic ions by the reducing carbohydrate and the

deposition of the ions in metallic form on the surface, thereby obtaining a metal-coated surface.

In a preferred embodiment, the metal is selected from the group consisting of silver, titanium, cobalt, nickel, copper, zinc, zirconium, molybdenum, tin, and lead. In a still more preferred embodiment, the metal is silver. In this case, the reaction between the salt of the silver ion and the reducing monosaccharide, which is attached to the substrate, is carried out by the Tollens' reaction. This reaction consists on the reduction of a complex containing silver ions and ammonia in the presence of the reducing monosaccharide. This results in the reduction of the diamminesilver(I) ions to metallic silver and the oxidation of the monosaccharide to the corresponding carboxylate.

Tollens' reaction is a multi-stage process, involving the oxidation of an aldehyde to the carboxylic acid, and reduction of aqueous silver ions to the metal. First, a silver diamine complex is prepared by reacting silver oxide (which is prepared by routine means from silver nitrate and sodium hydroxide) and ammonium hydroxide according to the following reaction:

$$Ag_2O + 4NH_4OH \rightarrow 2[Ag(NH_3)_2]^+ + 2OH^- + 3H_2O$$

The diamine complex is stable, between 4-24 h, in aqueous solution and can be stored until needed. The silver deposition process is initiated when the diamine complex is contacted with the reducing monosaccharide by reaction of the monosaccharide with hydroxyl ions in the alkali solution causing it to be oxidized to a carboxylic acid and releasing two electrons:

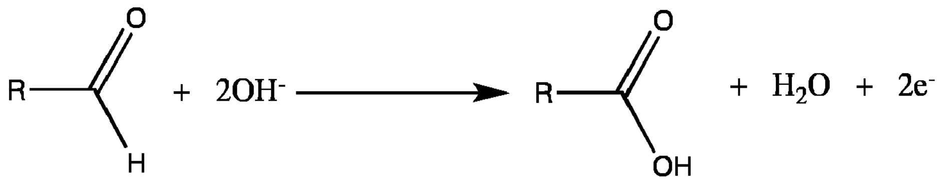

The electrons released by the oxidation of the aldehyde can each then reduce a silver diamine complex to give metallic silver and free ammonia:

$$[Ag(NH_3)_2]^+ + e- \rightarrow Ag + 2NH_3$$

This redox reaction can be summarized as:

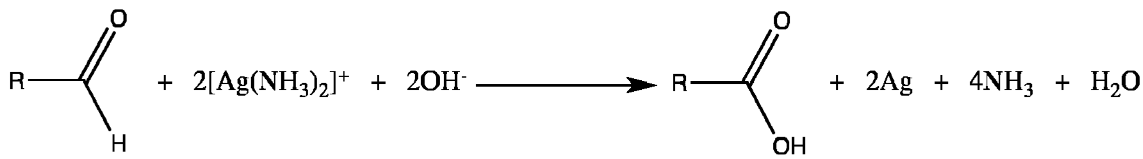

The reaction can be carried out for at least 5 minutes, at least 10 minutes, at least 15 minutes, at least 20 minutes, at least 30 minutes, at least 40 minutes, for at least 50 minutes, for at least 60 minutes, for at least 2 h, for at least 3 h, for at least 4 h, for at least 5 h, for at least 10 h or more.

The third step of the method of the invention is carried out until a deposit of sufficient density or thickness is formed. In a particular embodiment, a deposit of sufficient density or thickness is a deposit which is able to release an appropriate amount of the metallic ion. The released amount of the metallic ion can be quantified by any suitable method known by the skilled person. For example, if the metal ion is a silver ion, the released of silver ions can be quantified in a 50% nitric acid solution by optic inductively coupled plasma (ICP). An appropriate amount of the metallic ion is an amount which is sufficient to display antimicrobial activity without being toxic for cells, especially for human cells. In a particular embodiment, when the metallic ion is a silver ion, said amount is between 0,1 ppm and 10 ppm, more particular embodiment, said amount is between 0,1 ppm and 2 ppm.

The first method of the invention can be applied to one or more surfaces of the substrate, either to the whole surfaces or to only a portion of the surfaces.

A second surface of the substrate can be also contacted with a reactive monomer containing an activated carboxyl group under conditions adequate for the formation of a polymer coat containing said activated carboxyl group on the surface of the substrate formed by polymerization of the monomer. However, instead of reacting said second surface with a reducing carbohydrate containing a reactive group, the second surface is contacted with particles, selected from nanoparticles and microparticles, whose surface is modified with reactive groups, under conditions adequate for the formation of n covalent bond between the reactive group from the outside of the particle and the activated carboxyl

group on the surface of the substrate, thereby resulting in the modification of the second surface modified with particles and wherein the particle contains at least one therapeutically active compound. In this way, substrates are obtained wherein a first surface contains a metallic coat, preferably a silver coat and a second surface contains an active agent. These substrates are particularly useful for the constructions of stents wherein the external and internal surfaces are in contact with different body compartments and, accordingly, each surface requires specific active agents.

Therefore, in a preferred embodiment, the method of the invention is also applied to at least a second surface of the substrate, wherein said second surface is contacted with a particle modified with a group reactive with the activated carboxyl group, wherein said particle is selected from a nanoparticle and a microparticle, wherein said particle contains at least a therapeutically active compound, and wherein said contacting is carried out under conditions adequate for the formation of a covalent bond between the reactive group in the particle and the activated carboxyl group on the surface of the substrate, thereby resulting in the modification of the second surface with particles.

In a particular embodiment, the contacting with a particle can be performed on the whole surface of the second surface of the substrate or, alternatively, on only a part of the second surface. In another particular embodiment, one or more surfaces of the substrate can be modified with different coatings in different parts of the surfaces, for example, a portion of a surface of the substrate can be coated with a metallic ion and a different portion of the same surface of the substrate can be coated with particles comprising a therapeutically active compound; or a portion of a surface of the substrate can be coated with particles comprising a therapeutically active compound and another portion of the same surface can be coated with particles comprising the same or different therapeutically active compound. When different portions of the same surface of the substrate are going to be coated with different coatings, the portions that are not going to be coated with the first coating can be covered with a mask during the application of the first coating. Once the first portion of the surface is coated with the first coating, said first portion can be covered with a mask and the portions that are going to be coated with the second coating can be uncovered, so the second coated can be applied to these portions. In a particular embodiment, the group reactive with the activated carboxyl group is an amino group and the covalent bond between the reactive group in the particle and the activated carboxyl group on the surface of the substrate is an amide group.

In one embodiment, the polymeric coat of the first surface and the polymeric coat of the second surface are the same. In another embodiment, the polymeric coat of the first surface and the polymeric coat of the second surface are different. In the case that the polymeric coats are identical, both surfaces of the substrate can be coated simultaneously by contacting them with the reactive monomers. In the case that the polymeric coat of the first surface and the polymeric coat of the second surface are different, then the coating of the first surface has to be carried out under conditions wherein the second surface is not accessible to the reactive monomers forming the first layer. The substrate in which a first surface is coated is then contacted with the reactive monomers suitable for forming the polymeric layer on the second surface wherein the first coated surface is not accessible to the reactive monomers.

As used herein, the term "nanoparticle" refers to particles between about 1 nm and less than 1000 nm in diameter. In certain embodiments of the invention, the nanoparticle has a diameter of about 3 nm to about 1,000 nm. In certain embodiments, the nanoparticle has a diameter of about 3 nm to about 10 nm. In certain embodiments, the nanoparticle has a diameter of about 10 nm to about 1000 nm. In certain embodiments, the nanoparticle has a diameter of about 50 nm to about 1000 nm. In certain embodiments, the nanoparticle has a diameter of about 50 nm to about 500 nm. In certain embodiments, the nanoparticle has a diameter of about 50 nm to about 300 nm. In certain embodiments, the nanoparticle has a diameter of about 100 nm to about 300 nm. In certain embodiments, the nanoparticle has a diameter of about 150 nm to about 300 nm. In certain embodiments, the nanoparticle has a diameter of about 200 nm to about 250 nm. In certain embodiments, the nanoparticle has a diameter of about 100 nm to about 200 nm. In certain embodiments of the invention, the nanoparticle has a diameter of about 100 nm to about 150 nm.

The mean diameter of the nanoparticles of the present invention may be determined by methods known in the art, preferably by dynamic light scattering. In particular, the invention relates to nanoparticles that are solid particles with a diameter of from about 1 to about 1000nm when analyzed by dynamic light scattering at a scattering angle of 90° and at a temperature of 25°C, using a sample appropriately diluted with filtered water and a suitable instrument, such as the Zetasizer™ instruments from Malvern Instruments (UK) according to the standard test method ISO 22412:2008 (cumulants method A. 1.3.2). Where a particle is said to have a diameter of x nm, there will generally be a distribution of particles about this mean, but at least 50% by number (e.g. >60%, >70%, >80%, >90%, or more) of the particles will have a diameter within the range x±20%.

In another particular embodiment, the particle is a microparticle.

The term "microparticle", as used herein, refers to a particle having a diameter between 1 and 250 μm and including, without limitation, microspheres and microcapsules. In a preferred embodiment the compound is a microparticle.

The composition of the particles is not particularly limiting as long as they can encapsulate one or more of the therapeutically active compounds and as long as they can be modified to contain amino groups on the outer surface so as to allow the reaction with the activated carboxyl groups which form part of the polymeric coat applied to the second

surface.

In certain embodiments of the invention, the particle is a polymeric particle wherein the polymer is selected from poly-(lactic-co-glycolic acid), polyanhydride, polysulfonamide, alginate, chitosan, polyethyleneimine, polyethylene glycol, polyethylene glycol-polyester (PEG-polyester), poly-L-lysisne, polyglutamic acid, cellulosic derivatives (e.g., ethyl cellulose), and acrylic acid based polymers (e.g., hydroxypropyl methacrylamide). In certain embodiments, the polymer is polyethylene glycol-polyester (PEG-polyester).

The particles can be obtained by any suitable method known by the skilled person. In a particular embodiment, when the particle is a polymeric particle, it can be obtained by contacting the therapeutically active compound with the polymer under suitable conditions to form particles. In a more particular embodiment, the therapeutically active compound and the polymer may be mixed in a suitable watersoluble organic solvent at concentrations appropriate to obtain the desired ratio and added dropwise to a continuously stirred solution. The resulting particles may be further purified by means of dialysis, filtration or centrifugation (Di Mauro, P. P., & Borrós, S. Pharmaceutical Research 2014, (10)).

The particles are modified in order to present a group reactive with the activated carboxyl group on the outside of the particle surface. Said reactive groups have been previously defined. In a preferred embodiment, the group reactive with the activated carboxyl group is an amino group. It will be understood that the reactive group, preferably the amino group, may be directly linked to the atoms forming part of the nanoparticle shell or can be contacted to the nanoparticle by means of a linker region. For example, the linker can be between 4 and 15 atoms (i.e., 4, 5, 6, 7, 8, 9, 10, 11, 12, 13, 14, or 15) long.

Numerous biologically active agents, not necessarily exclusive of those listed above, can be encapsulated in the nanoparticles used in the method according to the invention.

In one embodiment, the therapeutically active compound is an antiproliferative compound. Suitable antineoplastic/antiproliferative/anti-mitotic agents are antimetabolites such as purine analogs (e.g., 6-mercaptopurine or cladribine, which is a chlorinated purine nucleoside analog), pyrimidine analogs (e.g., cytarabine and 5-fluorouracil) and methotrexate, nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin, cerivastatin, flavopiridol and suramin.

In one embodiment, the therapeutically active compound is an anti-proliferative and anti-migration compound such as cytochalasin.

In one embodiment, the therapeutically active compound is an anti-angiogenic compound. Suitable anti-angiogenic agents are antibody or other antagonist to an angiogenic agent, e.g., fusion proteins that binds to VEGF A such as ZALTRAP, (Aflibercept), antibodies to VEGF-A such as AVASTIN(R) (bevacizumab) or to the VEGF-A receptor (e.g., KDR receptor or Flt-1 receptor), anti PDGFR inhibitors such as GLEEVEC(R) (Imatinib Mesylate), small molecules that block VEGF receptor signaling (e.g., PTK787/ZK2284, SU6668, SUTENT@/SU1 1248 (sunitinib malate), AMG706, or those described in, e.g., international patent application WO 2004/113304).. See, e.g., Klagsbrun and D'Amore, Annu. Rev. Physiol. 1991, 53:217-39; Streit and Detmar, Oncogene 2003, 22:3172-3179 (e.g., Table 3 listing anti angiogenic therapy in malignant melanoma); Ferrara and Alitalo, Nature Medicine 1999, 5(12):1359-1364; Tonini et al., Oncogene 2003, 22:6549-6556 (e.g., Table 2 listing known anti-angiogenic factors); and, Sato, Int. J Clin. Oncol.2003, 8:200-206 (e.g., Table 1 listing anti-angiogenic agents used in clinical trials).

In one embodiment, the therapeutically active compound is an anti-inflammatory compound. Suitable anti-inflammatory agents are dexamethasone, prednisolone, corticosterone, budesonide, estrogen, sulfasalazine and mesalamine.

In one embodiment, the therapeutically active compound is an anti-inflammatory compound. Suitable anti-inflammatory agents include aspirin, sodium salicylate, sulpyrine, indomethacin, diclofenacsodium, loxoprofensodium, ferbinac, zaltoprofen, piroxicam, nimesulide, meloxicam, celexicob, tialamide, emorfazone, buprenorphine, eptazocine hydrobromide, pentazocine, butorphanol tartrate, tramadol hydrochloride, ketorolac, meperidine hydrochloride, morphine hydrochloride, morphine sulfate, hydromorphine, fentanyl citrate, fentanyl, and mofezolac.

In one embodiment, the therapeutically active compound is a cytotoxic compound. Suitable cytotoxic compounds include methotrexate, cyclophosphamide, and thalidomide, as well as metabolites, salts, polymorphs, prodrugs, analogues, and derivatives of methotrexate, cyclophosphamide, and thalidomide. In certain embodiments, preferred cytotoxic small molecules are the pharmaceutically active metabolites of cytotoxic agents. For example, in the case of cyclophosphamide, preferred metabolites are pharmaceutically active metabolites of cyclophosphamide, including but not limited to 4-hydroxycyclophosphamide, aldophosphamide, phosphoramide mustard, and combinations thereof.

In one embodiment, the therapeutically active compound is an anti-thrombotic active agent. Suitable anti-thrombotic active agent include platelet adhesion inhibitors, such as albumin and polyethylene oxide, platelet aggregation inhibitors including cilostazole, aspirin and thienopyridine (ticlopidine, clopidogrel) and GP IIb/IIIa inhibitors, such as abciximab, epitifibatide and tirofiban, coagulation pathway modulators including heparinoids, such as heparin, low molecular weight heparin, dextran sulfate and beta-cyclodextrin tetradecasulfate and thrombin inhibitors, such as hirudin,

hirulog, PPACK(D-phe-L-propyl-L-arg-chloromethylketone) and argatroban, FXa inhibitors, such as antistatin and TAP (tick anticoagulant peptide), Vitamin K inhibitors, such as warfarin, as well as activated protein C.

In a preferred embodiment, the particles contain an anti-proliferative agent. In a more preferred embodiment, the anti-proliferative agent is placlitaxel. Suitable nanoparticles capable of encapsulating paclitaxel have been described by Ma P. et al., (, J. Nanomed. Nanotechol. 2013, 4-2) and include, without limitation, PLGA (Poly[Lactic-Co-Glycolic Acid), PCL (Poly [ε-Caprolactone]), PLA (Poly[L-Lactide]), chitosan, gelatin, hyaluronic acid, PBCA (Poly[Butyl Cyanoacrylate]), albumin , HPG (Hyperbranched Polyglycerol) and PEG-PE (PEG-phosphatidylethanolamine).

In a preferred embodiment, the reaction is carried out until a sufficient amount of paclitaxel is associated with the surface. The amount of paclitaxel associated to the particles modifying the surface of the substrate can be quantified by any suitable technique known by the skilled person, for example by means of the quantification technique described in the examples below.

Without wanting to be bound by any particular theory, it is believed that the particles containing paclitaxel suffer from degradation resulting in the controlled released of paclitaxel.

*Method for modifying the surface of a substrate with a particle*

The first step of the first method for can be also used for modifying the surface of a substrate with a particle containing a therapeutically active compound. Once the first step of the first method of the invention is applied resulting in the surface of the substrate covered by polymerized reactive monomer, the substrate can be further modified by a particle modified with a reactive group and containing the therapeutically active compound.

Therefore, in a second aspect, the invention relates to a method for modifying the surface of a substrate with a particle selected from a nanoparticle and a microparticle, hereinafter second method of the invention, comprising the steps of

(i) contacting at least a first surface the substrate with a reactive monomer containing an activated carboxyl group under conditions adequate for the formation of a polymer coat containing said activated carboxyl group on the surface of the substrate by polymerization of the monomer and

(ii) contacting the substrate obtained in step (i) with a particle modified with a group reactive with said activated carboxyl group, wherein said particle is selected from a nanoparticle and a microparticle, wherein said particle contains at least a therapeutically active compound, and wherein said contacting is carried out under conditions adequate for the formation of a covalent bond between the reactive group in the nanoparticle and the activated carboxyl group on the surface of the substrate, thereby resulting in the modification of the second surface with nanoparticles.

All the above- mentioned definitions regarding the terms included in the first step of the firs method of the invention, as well as its particular and preferred embodiments, are fully applicable to the second method for the invention.

The terms "particle", "nanoparticle", "microparticle", "therapeutically active compound" and "group reactive with the activated carboxyl group", as well as their particular and preferred embodiments, have been previously defined in connection to the first method of the invention and are fully applicable to the second method of the invention.

In a particular embodiment of the second method of the invention, at least a second surface of the substrate is coated with a metallic ion by using the first method of the invention.

In one embodiment, the polymeric coat of the first surface and the polymeric coat of the second surface are the same. In another embodiment, the polymeric coat of the first surface and the polymeric coat of the second surface are different. In the case that the polymeric coats are identical, both surfaces of the substrate can be coated simultaneously by contacting them with the reactive monomers. In the case that the polymeric coat of the first surface and the polymeric coat of the second surface are different, then the coating of the first surface has to be carried out under conditions wherein the second surface is not accessible to the reactive monomers forming the first layer. The substrate in which a first surface is coated is then contacted with the reactive monomers suitable for forming the polymeric layer on the second surface wherein the first coated surface is not accessible to the reactive monomers.

In a particular embodiment, the contacting with a particle can be performed on the whole surface of one or more surfaces of the substrate or, alternatively, on only a portion of one or more surfaces. In another particular embodiment, one or more surfaces of the substrate can be modified with different coatings in different parts of the surfaces, for example, a portion of a surface of the substrate can be coated with a metallic ion and a different portion of the same surface of the substrate can be coated with particles comprising a therapeutically active compound; or a portion of a surface of the substrate can be coated with particles comprising a therapeutically active compound and another portion of the same surface can be coated with particles comprising the same or different therapeutically active compound.

*Coated substrate of the invention*

In another aspect, the invention relates to a substrate, hereinafter first substrate of the invention, which is obtainable by the first method of the invention.

In one embodiment, the substrate contains a silicone-based polymeric substrate, more particular polydimethylsiloxane. In another embodiment, the substrate has been obtained using an activated methacrylate as reactive monomer containing a reactive carboxyl group. In yet another embodiment, the activating group used in the activated methacrylate is a pentafluoremethyl group. In another embodiment, the substrate is coated by a polymer layer produced by chemical vapor deposition, preferably, plasma-enhanced chemical vapor deposition (PECVD). In another embodiment, the metal, which coats the substrate is silver. In another embodiment, the silver coat has been generated by the Tollens' reaction using diamine-silver (I) complex ions. In another embodiment, the substrate has been obtained using glucosamine as reducing carbohydrate.

In another embodiment, the first surface of the substrate contains a metal coat generated according to the first method of the invention and a second surface of the substrate, which is coated with particles selected from nanoparticles and microparticles, wherein said particles contain at least one therapeutically active compound, obtained by reacting the reactive carboxyl group forming part of the polymeric coat with particles containing said therapeutically active compound and having groups reactive with said carboxyl group on their surface. In one embodiment, the therapeutically active compound forming part of the particles attached to the second surface is selected from the group consisting of an antiproliferative compound, an anti-migration compound, an antiangiogenic compound, an anti-inflammatory compound, a cytostatic compound, a cytotoxic compound, an antithrombotic active agent or a combination of two or more of the above.

In a particular embodiment, one or more surfaces of the substrate are coated by the metal coat generated according to the first method of the invention, being said metal coat coating the whole surfaces or only a portion of the surfaces. In another particular embodiment, one or more surfaces of the substrate are covered with different coatings in different portions of the surfaces, for example, a portion of a surface of the substrate is coated with a metallic ion and a different portion of the same surface of the substrate is coated with particles comprising a therapeutically active compound.

In another aspect, the invention relates to a substrate, preferably a silicone-based polymer, which contains:

(i) An internal coat of a polymeric material, said polymer being modified with an oxidized monosaccharide connected to the polymeric layer formed between carboxyl groups in the layer and groups reactive with said carboxyl groups in the carbohydrate and
(ii) an external coat of a metal produced by reduction of ions of said metal.

It will be understood that the first layer is the result of the polymerization of the reactive monomer containing activated carboxyl group under conditions adequate for the polymerization of the monomer and the formation of the layer, followed by reaction with a reducing carbohydrate, preferably a reducing monosaccharide, containing a group reactive with the carboxyl group, preferably an amino group. The group reactive with the carboxyl group reacts with the activated carboxyl group, forming a covalent bond and releasing the protecting group. Accordingly, the protecting group does not form part of the final coated substrate.

The second layer is the result of the deposition of the metal caused by the reducing carbohydrate. This reaction leads to the reduction of the metal concomitantly with the oxidation of the reducing carbohydrate, which is then converted to the corresponding carboxylate. Accordingly, the carbohydrate appears in the coated substrate not in the reduced form (as aldose or ketose), but rather in its oxidized form.

It will be understood that the stent is made of any material. In a particular embodiment, the stent is made ofa polymeric material, preferably a silicone-based polymeric material, which has the appropriate flexibility/rigidity, tear resistance, and sterilization resistance for use in the devices of the invention. In another particular embodiment, the stent is made of a metallic material. In this particular embodiment, the metallic material can be covered by a layer of a polymeric material, preferably a silicone-based polymeric material.

In one embodiment, the entire surface of the substrate is modified by a coat comprising a first polymeric coat, which is closer to the surface of the substrate, and a second metallic coat, which is farther from the surface of the substrate. In another embodiment, a first surface of the substrate is modified by an internal polymeric coat and an external metallic coat and a second surface of the substrate is modified by a polymeric coat which is coupled to particles selected from nanoparticles and microparticles containing a therapeutically active compound encapsulated within, said particles being connected to the polymeric layer by covalent bonds between carboxyl groups in the polymeric layer and groups reactive with said carboxyl groups on the surface of the particles. In one embodiment, the polymeric coat of the first surface and the polymeric coat of the second surface are the same. In another embodiment, the polymeric coat of the first surface and the polymeric coat of the second surface are different.

In a preferred embodiment, the substrate is a stent. As used herein, the term stent refers to a medical device configured for use within a bodily structure, such as within a body lumen. The term "stent" is intended to include, but is not limited to, self-expandable stents, balloon-expandable stents, stent- grafts, and grafts. In a still more preferred embodiment, the stent is a coronary stent, a vascular stent, a tracheal stent, a bronchial stent, a urethral stent, an esophageal stent, a biliary stent, a renal stent, a stent for use in the small intestine, a stent for use in the large intestine, a laryngeal implant, a bypass, a catheter or an ileostomy

In one embodiment, the stent is tubular in shape. In this case, the silicone-based polymeric substrate may be provided as a sheet, which is then coated according to the methods of the present invention and then shaped in order to provide it with a tubular shape. Alternatively, the silicone-based polymeric substrate may be shaped to a tubular shape and then the internal and external surfaces of the tube coated according to the methods of the invention. In the latter case, a solution containing the monomer capable of forming the silicone-based polymer is cast on a rod- shaped mold to form a thin film around the mold, with subsequent drying of the solution and removal of the mold to form the tubular stent.

In a more preferred embodiment, the stent is a tracheal stent, in which case the internal surface contains the metal coat. In a more preferred embodiment, the internal surface contains the metal coat and the external coat contains the nanoparticles containing the therapeutic active compound. In a still more preferred embodiment, the internal coat contains silver as metal and the external coat contains paclitaxel encapsulated in the nanoparticles.The methods according to the invention are adapted for coating for example endoprostheses and in particular stents such as for example coronary stents, vascular stents, tracheal stents, bronchial stents, urethral stents, esophageal stents, biliary stents, renal stents, stents for use in the small intestine, stents for use in the large intestine. Moreover, guiding wires, helices, catheters, cannulas, tubes as well as generally tubular implants or parts of the above mentioned medical devices can be coated according to the invention provided that a structural element comparable to a stent is contained in such medical device. As far as expandable medical devices or respectively endoprostheses are used, the coating preferably is carried out during the expanded state of the respective device.

The coated medical devices are preferably used for maintaining patency of any tubular structure, for example the urinary tract, esophagus, trachea, the biliary tract, the renal tract, duodenum, pilorus, the small and the large intestine, blood vessels in the whole body including brain, but also for maintaining the patency of artificial openings such as used for the colon or the trachea.

In another aspect, the stent is used in the prevention, reduction or treatment of stenosis, restenosis, arteriosclerosis, atherosclerosis, vessel occlusions, vessel constrictions, aneurysms, and for artificial openings and accesses.

In another aspect, the invention relates to a substrate, hereinafter second substrate of the invention, which is obtainable by the second method of the invention.

In one embodiment, the substrate contains a silicone-based polymeric substrate, more particular polydimethylsiloxane. In another embodiment, the substrate has been obtained using an activated methacrylate as reactive monomer containing a reactive carboxyl group. In yet another embodiment, the activating group used in the activated methacrylate is a pentafluoremethyl group. In another embodiment, the substrate is coated by a polymer layer produced by chemical vapor deposition, preferably, plasma-enhanced chemical vapor deposition (PECVD). In another embodiment, the particle is a polyethylene glycol-polyester particle. In one embodiment, the therapeutically active compound forming part of the particles attached to the surface of the substrate is selected from the group consisting of an antiproliferative compound, an anti-migration compound, an antiangiogenic compound, an anti-inflammatory compound, a cytostatic compound, a cytotoxic compound, an antithrombotic active agent or a combination of two or more of the above.

In another aspect, the invention relates to a substrate, preferably a silicone-based polymer, which contains a coat of a polymeric material, said polymer being coupled to particles selected from nanoparticles and microparticles containing a therapeutically active compound encapsulated within, said particles being connected to the polymeric layer by covalent bonds between carboxyl groups in the polymeric layer and groups reactive with said carboxyl groups on the surface of the particles. It will be understood that the type of bond between the polymeric coat and the nanoparticles will depend on the nature of the group reactive with an activated carboxyl group found in the nanoparticles. In a preferred embodiment, the group reactive with an activated carboxyl group is an amine group, which will result in that the nanoparticles and the polymeric layer are connected by an amide bond.

The polymeric material has been previously defined in connection with the first substrate of the invention.

In one embodiment, the entire surface of the substrate is modified by a polymeric coat which is coupled to particles containing a therapeutically active compound encapsulated within, said particles being connected to the polymeric layer by covalent bonds formed between carboxyl groups in the polymeric layer and groups reactive with said carboxyl groups on the surface of the particle, preferably amide bonds if the groups reactive with said carboxyl groups are amine groups.

In a particular embodiment, one or more surfaces of the substrate are coated by a polymeric coat which is coupled to particles containing a therapeutically active compound, generated according to the second method of the invention, being said polymeric coat coupled to nanoparticles coating the whole surfaces or only a portion of the surfaces. In another particular embodiment, one or more surfaces of the substrate are covered with different coatings in different

portions of the surfaces, for example, a portion of a surface of the substrate is coated with a metallic ion and a different portion of the same surface of the substrate is coated with particles comprising a therapeutically active compound, or a portion of a surface of the substrate is coated with particles comprising a therapeutically active compound and a different portion of the same surface of the substrate is coated with particles comprising the same or different therapeutically active compound.

In one embodiment, the first and second surfaces of the substrate are modified by an internal polymeric coat and an external metallic coat as described in connection with the first method of the invention.

In another embodiment, the first and second surfaces of the substrate are modified by a polymeric coat which is coupled to particles containing a therapeutically active compound encapsulated within, said particles being connected to the polymeric layer by covalent bonds between carboxyl groups in the polymeric layer and groups reactive with said carboxyl groups on the surface of the particle. In one embodiment, the particles which coat the first and second surfaces contain the same therapeutically active compound. In another embodiment, the particles which coat the first and second surfaces contain the different therapeutically active compounds.

In another embodiment, a first surface of the substrate is modified by a polymeric coat which is coupled to particles containing a therapeutically active compound encapsulated within, said particles being connected to the polymeric layer by covalent bonds between carboxyl groups in the polymeric layer and groups reactive with said carboxyl groups on the surface of the particle, and a second surface of the substrate is modified by an internal polymeric coat and an external metallic coat as described in connection with the first substrate of the invention. In one embodiment, the polymeric coat of the first surface and the polymeric coat of the second surface are the same. In another embodiment, the polymeric coat of the first surface and the polymeric coat of the second surface are different.

When the substrate is a tracheal stent, it may be advantageous having a portion of the outer surface of the stent covered by a polymeric material coupled to particles comprising a therapeutically active compound, such a paclitaxel. In this particular embodiment, said particle-coupled polymeric material may be present only in the central portion of the outer surface (i.e. not covering the distal regions of the stent). In another embodiment of the tracheal stent, the inner surface is coated with polymeric material containing a metallic layer obtained according to the first methof of the invention and the outer surface is coated in the central region by a polymeric material coupled to particles comprising a therapeutically active compound, such a paclitaxel.

In a preferred embodiment, the substrate is a stent. The term "stent" ant its particular and preferred embodiments have been defined in connection with the first substrate of the invention.

*Further aspects of the invention*

1. A method for coating a substrate with a metallic ion comprising the steps of

   (i) contacting at least a first surface of the substrate with a reactive monomer containing an activated carboxyl group under conditions adequate for the formation of a polymer coat containing said activated carboxyl group on the surface of the substrate by polymerization of the monomer,
   (ii) contacting the substrate obtained in step (i) with a reducing carbohydrate comprising a group reactive with said activated carboxyl group under conditions adequate for the formation of a covalent bond between the reactive group in the carbohydrate and the activated carboxyl group on the surface of the substrate, thereby obtaining an surface modified with a reducing carbohydrate and
   (iii) contacting the substrate obtained in step (ii) with a salt of said metallic ion under conditions adequate for the reduction of the metallic ions by the reducing carbohydrate and the deposition of the ions in metallic form on the surface, thereby obtaining a metal-coated surface.

2. The method according to aspect 1 wherein the substrate is a silicone-based polymer.

3. The method according to aspect 2 wherein the silicone-based polymer is polydimethylsilo xane.

4. The method according to any of aspects 1 to 3 wherein the group reactive with the activated carboxyl group is an amino group and wherein the covalent bond between the amino group in the carbohydrate and the activated carboxyl group on the surface of the substrate is an amide bond.

5. The method according to any of aspects 1 to 4 wherein the reactive monomer containing a reactive carboxyl group is an activated methacrylate.

6. The method according to aspect 5 wherein the activating group is a pentafluoremethyl group.

7. The method according to any of aspects 1 to 6 wherein the contacting in step (i) is carried out using chemical vapor deposition.

8. The method according to aspect 7 wherein the chemical vapor deposition is plasma-enhanced chemical vapor deposition (PECVD).

9. The method according to any of aspects 1 to 8 wherein the metallic ion is a silver ion.

10. The method according to aspect 9 wherein the silver ion is provided as diamine-silver (I) complex ion.

11. The method according to aspect 10 wherein the diamine-silver (I) complex is ammonium silver nitrate.

12. The method according to any of aspect 1 to 11 wherein the reducing carbohydrate comprising a reactive group is N-glucosamine.

13. The method according to any of aspects 1 to 12 wherein step (i) is also applied to at least a second surface of the substrate and wherein said second surface is contacted with a particle modified with a group reactive with the activated carboxyl group, wherein said particle is selected from a nanoparticle and a microparticle, wherein said particle contains at least a therapeutically active compound, and wherein said contacting is carried out under conditions adequate for the formation of a covalent bond between the reactive group in the particle and the activated carboxyl group on the surface of the substrate, thereby resulting in the modification of the second surface modified with particles.

14. The method according to aspect 13 wherein the therapeutically active compound is selected from the group consisting of an antiproliferative compound, an anti-migration compound, an antiangiogenic compound, an anti-inflammatory compound, a cytostatic compound, a cytotoxic compound, an antithrombotic active agent and a combination of one or more of the above.

15. A method for modifying the surface of a substrate with a particle selected from a nanoparticle and a microparticle comprising the steps of

(i) contacting at least a first surface of the substrate with a reactive monomer containing an activated carboxyl group under conditions adequate for the formation of a polymer coat containing said activated carboxyl group on the surface of the substrate by polymerization of the monomer and
(ii) contacting the substrate obtained in step (i) with a particle modified with a group reactive with said activated carboxyl group, wherein said particle is selected from a nanoparticle and a microparticle, wherein said particle contains at least a therapeutically active compound, and wherein said contacting is carried out under conditions adequate for the formation of a covalent bond between the reactive group in the particle and the activated carboxyl group on the surface of the substrate, thereby resulting in the modification of the second surface with particles.

16. The method according to aspect 15 wherein the substrate is a silicone-based polymer.

17. The method according to aspect 16 wherein the silicone-based polymer is polydimethylsilo xane.

18. The method according to any of aspect 15 to 17 wherein the reactive monomer containing a reactive carboxyl group is an activated methacrylate.

19. The method according to aspect 18 wherein the activating group is a pentafluoremethyl group.

20. The method according to any of aspects 15 to 19 wherein the contacting in step (i) is carried out using chemical vapor deposition.

21. The method according to aspect 20 wherein the chemical vapor deposition is plasma-enhanced chemical vapor deposition (PECVD).

22. The method according to any of aspects 15 to 21 wherein the particle is a polyethylene glycol-polyester particle.

23. The method according to any of aspects 15 to 22 wherein the group reactive with the activated carboxyl group is an amino group and wherein the covalent bond between the amino group in the particle and the activated carboxyl group on the surface of the substrate is an amide bond.

24. The method according to any of aspects 15 to 23 wherein the therapeutically active compound is selected from the group consisting of an antiproliferative compound, an anti-migration compound, an antiangiogenic compound, an anti-inflammatory compound, a cytostatic compound, a cytotoxic compound, an antithrombotic active agent and a combination of one or more of the above.

25. A substrate obtainable by a method according to any of aspects 1 to 24.

26. The substrate according to aspect 25, which is a stent.

27. The stent according to aspect 26, wherein a first surface has been coated with a metallic coat and wherein a second surface has been coated with particles, and wherein the surface containing the metallic coat is the inner surface and the surface containing the particle coat is the external surface.

28. The stent according to any of aspects 25 to 27 which is a coronary stent, a vascular stent, a tracheal stent, a bronchial stent, a urethral stent, an esophageal stent, a biliary stent, a renal stent, a stent for use in the small intestine, a stent for use in the large intestine, a laryngeal implant, a bypass, a catheter or an ileostomy.

29. The stent according to aspect 28 for use in the prevention, reduction or treatment of stenosis, restenosis, arteriosclerosis, atherosclerosis, vessel occlusions, vessel constrictions, aneurysms, and for artificial openings and accesses.

The invention is described below by way of the following examples, which are to be construed as merely illustrative and not limitative of the scope of protection.

EXAMPLES

*Materials*

Argon 5.0 and Oxygen 5.0 were purchased from Abelló Linde S.A. (Spain). PFM was purchased from Apollo ScientificLTD (UK). D-(+)-glucosamine hydrochloride, decylamine and silver nitrate were purchased from Sigma Aldrich.

*Substrates*

The substrates used in this work were Si-wafers and polydimethylsiloxane (PDMS) samples. Sylgard® 184 kit (Dowcorning, USA) was used to fabricate PDMS plates. The kit (10:1) was spread by a paint applicator to obtain plates of 500 μm thickness. These plates were incubated overnight at 70°C. The PDMS films were cut in circular shapes of 21 and 10 mm. It is important to denote that due to the high hydrophobicity and electrostatic charge of PDMS, samples need an accurate washing treatment. PDMS plates were washed with Sodium Dodecyl Sulphate (SDS, Sigma Aldrich) 5%, rinsed with milliQ water, washed with Hellmanex® (Helma® Analytics, Germany) solution 2%, rinsed again with milliQ water and finally with ethanol (Scharlab, Spain). Si-wafers were washed with the same protocol as PDMS plates. Then, samples were dried in a stream of nitrogen and stored in Petri dishes from Nunc LabTek™.

*Plasma Reactors*

The surface modifications carried out in this work were performed using stainless steal vertical plasma reactor. This reactor consists of a stainless steal chamber (diameter: 25.5 cm, length: 41.6 cm) vertical plate reactor). The ground electrode is the reactor chamber and the RF (Radio Frequency) electrode is an aluminium plate, which is used to hold the samples for polymerization. Additionally, the RF electrode is connected to a RF pulse generator (13.56 MHz) via a matching box. Gases and monomers are supplied via a standard manifold with gas fluxes adjusted with a tree of needle valves. The system pressure is monitored using a vacuum gauge controller (MKS PDR900, USA) connected with a cold cathode/micropirani vacuum transducer (MKS 972 DualMag, USA) positioned at the centre of the reactor. The system has a nitrogen cold trap and a chemical trap filled with active carbon connected to avoid non-reacted monomer to reach

the pump (Trivac D 16BCS/PFPE Leybold, Germany). The typical base pressure to all experiments is $6 \cdot 10^{-4}$ mbar and the monomer vapour (PFM) was introduced at a constant pressure to 0.02-0.04 mbar.

*Plasma Polymerization*

Substrates were placed on an aluminium plate in the centre of the reactor. Before introduction of the substrate, the chamber was cleaned in continuous wave $O_2$/Ar (1:1) plasma for approximately 1 h at a power of 150 W. The continuous radio frequency power was fixed at 15 W, and pulsed plasma polymerization (duty cycle 10/20) was carried out for 3-5 min. Plasma discharge was then turned off, and the PFM vapour flow was kept constant for additional 3 min. After the polymerization process, samples (pPFM) were carefully removed from the reaction chamber and stored until further use under argon atmosphere.

*Modified Surface Characterization*

The modified surfaces were characterized through water contact angle (DSA100, Krüss; WCA), Time of Flight (ToF-SIMS) and Ultra High Resolution SEM - UHRSEM (NovaNanoSEM 230, FEI) with high voltage. To analyze the surface modification by WCA and AFM, both modified PDMS samples and Si-wafers were directly analyzed after polymerization. The TOF-SIMS analyses were performed using a TOF-SIMS IV (ION-TOF, Munster, Germany) operated at a pressure of $5 \times 10^{-9}$ mbar. Samples were bombarded with a pulsed Bismuth liquid metal ion source ($Bi3^{++}$), at energy of 25 keV. The gun was operated with a 20 ns pulse width, 0.3 pA pulsed ion current for a dosage lower than $5 \times 10^{11}$ ions/$cm^2$, well below the threshold level of 1x1013 ions/$cm^2$ generally accepted for static SIMS conditions. Secondary ions were detected with a reflector time-of-flight analyzer, a multichannel plate (MCPs), and a time-to-digital converter (TDC). Measurements were performed with a typical acquisition time of 10 s, at a TDC time resolution of 200 ps and 100 us cycle time. Charge neutralization was achieved with a low energy (20 eV) electron flood gun. Secondary ion spectra were acquired from a randomly rastered surface areas of 100 μm x 100 μm within the sample's surface. Secondary ions are extracted with 2 kV voltages and are post-accelerated to 10 keV kinetic energy just before hitting the detector. Mass spectral acquisition was performed within the ION-TOF Ion Spec software (version 4.1).

*Silver deposition*

In order to achieve the antimicrobial coating, the silver was reduced by a reductive sugar using the Tollens' method. This method consists on the complexation of the silver ions with ammonia and its subsequent reduction using a reductive sugar on a heated bath. By using an immobilized sugar, the diaminesilver (I) ions will be reduced to metallic silver resulting in a surface coating. The glucosamine solution was prepared by dissolving glucosamine hydrochloride in milliQ water at 1 M. Then, pH was adjusted to 7.4 with concentrate sodium hydroxide. The reaction between glucosamine and the modified surface was performed incubating the modified samples in a glucosamine solution for 6 h. Tollens' reagent was prepared by adding 25 μl of ammonia 15% to 1 ml of silver nitrate 0.1 M, observing the precipitation of the silver oxide. Then, another 25 μl of ammonia 15% was added observing the complete dissolution of the precipitate due to the complexation of the silver by ammonia. The modified samples with the attached glucosamine were introduced on a vial with 4 ml of milliQ water. Then, 1 ml of the Tollens' reagent was added and heated on a water bath at 90°C for the desired time of reaction, 60 min. Silver was quantified using an ICP-OES (Optima 2100 DV, Perkin Elmer).

*Nanoparticle immobilization*

Nanoparticle immobilization was carried out using a suspension of nanoparticles 20 mg/ml. The pH of the suspension was adjusted to 7.4 with concentrate sodium hydroxide. The immobilization reaction of nanoparticles was performed incubating PFM modified PDMS sample in a nanoparticles suspension for 6 h. After the incubation process, samples were washed exhaustively with milliQ water and dried with compressed air for further use.

*Silver Release*

The Ag ion release of the coated samples was measured with ICP-OES. Different coated samples were immersed in 5 ml of TSB and in 5 ml of milliQ at 37 °C. After the desired time, 4 ml of each solution were extracted for analysis and replaced with 4 ml of fresh solution. The solutions were replenished and analyzed each day for 20 days.

*Immobilized Nanoparticle loaded with Paclitaxel Quantification*

Dried PDMS samples with immobilized nanoparticles loaded with paclitaxel were dissolved in acetonitrile and

the amount of entrapped drug was detected using an Ultra Performance Liquid Chromatography (UPLC) (Waters ACQUITY UPLC H-Class). A reverse phase BEH C18 column (1.7 μm 2.1 x 50 mm) was used. The mobile phase consisted in a mixture (65:35 v/v) of mobile phase A (water, containing 0.1% of acetic acid) and mobile phase B (methanol, containing a 0.1% of acetic acid) and was delivered at a flow rate of 0.6 ml/min. A 1.0 μl aliquot was injected into de UPLC system, and total analytical run time was 3 min. Paclitaxel and docetaxel as internal standard (I.S.) were quantified by UV detection (λ=227 nm, Waters TUV detector). A calibration curve of standard drug solution (paclitaxel) and I.S. was used to obtain the calibration solutions, which was linear over the range of 0.1 - 2.5 μg/ml of paclitaxel (with 6 μg/ml of docetaxel) with a correlation coefficient of $R^2$ = 0.995. Drug immobilization was calculated using Equation 1.

**Equation 1.** Drug Immobilization

$$Drug\ immobilization\left(\frac{ng}{mm^2}\right)=\frac{Mass\ of\ drug\ in\ sample}{Area\ of\ sample}$$

*Bacterial in vitro Assay*

Bacterial cultures were performed at Hospital Bellvitge in Barcelona using commercial *Pseudomonas aeruginosa* (PA01) strain and clinical *P. aeruginosa* (282939) and *Staphylococcus aureus* (329531) strains. Bacteria were seeded in TSB (Tryptic Soy Broth) medium to obtain 10 ml of $10^8$ cfu / ml and agitated at 37 °C overnight. After incubation, 20 ml of bacteria suspensions at $10^7$ and $10^6$ cfu / ml were prepared by dilution. PDMS samples (modified with silver and non-modified) were placed on a sterile 24 well plate and 1 ml of each bacterial suspension was added. The plate with the samples was incubated at 37 °C without agitation for 24 h. After the incubation time, the supernatant of each well was aspirated and the samples were washed 7 times in milliQ water. Subsequently, samples were placed in a sterile 24 well plate and a Live&Dead® (Invitrogen) solution was added (2 ml of 0.9% NaCl + 3 μl of Solution A + 3 μl of Solution B) to each well. The 24 well plate was covered from light and incubated for 15 min. After incubation time, samples were washed with milliQ water and their fluorescence was observed on a confocal microscope; images of the double-labelled sections were acquired using a Leica TCS-SL filter-free spectral confocal laser scanning microscope (Leica Microsystems, Mannheim, Germany) equipped with a 488 nm argon laser, 543 nm and 633 nm He/Ne lasers (Centres Cientifics i Tecnològics, Universitat de Barcelona, Barcelona, Spain) using a 63x oil immersion objective (1.4 numerical aperture), image resolution of 1024 x 1024 pixels.

*Cell viability*

In order to evaluate the cytotoxicity of the drug loaded nanoparticles, Human Lung Normal Fibroblast, 6 weeks gestation (ATCC, CCL-186) cells were grown in EMEM (Gibco, Life Technologies) supplemented with 10% FBS, 5% Penicillin/Streptomycin and Amphotericin (2.5 μg/ml) and stenotic fibroblast (sample extracted by surgical biopsy) cells were grown in DMEM (Gibco, Life Technologies) supplemented with 10% FBS, 5% Penicillin/Streptomycin and Amphotericin (2.5 μg/ml) were used. Cells were plated in 96-well plates at a cellular density of 1000 cells/well. After 24h, medium was aspirated and substituted with medium with various paclitaxel concentrations (10 nM, 14 nM and 20 nM) of free drug, 1% and 3% theoretical drug loaded nanoparticles. One column, cells with non-encapsulated paclitaxel at the same range of concentrations (10-20 nM), was used as positive control; one column, cells without nanoparticles and without paclitaxel, was used as the control; another column, without cells, was used as the blank. After 1, 4, 8 and 12 days, cell viability was assessed via MTT (Quick Cell Proliferation Assay Kit II, JM-K302-500, MBL International Corporation, USA) following the manufacturer instructions. Briefly, cells were washed with PBS and incubated with a solution of the MTT reagent and complete media at 37°C. The absorption, which represented cell viability, was measured via microplate reader (Multiskan Ex, Thermo Scientific) at 450 nm. Cell viability was expressed as percentage of viable cells compared to control cells.

EXAMPLE 1

ToF-SIMS

In **Figure 1** positive and negative mass fragments of the pPFM coatings onto PDMS, and its subsequent reactions with glucosamine and finally the reduced silver through glucosamine can be observed. The non-modified

PDMS samples show the silicon mass fragment, m/z = 28, and additional PDMS typical peaks $-SiCH_3^+$, $CH_3SiO^-$, $SiO_2^-$, $SiC_3H_9^+$, $CH_3SiO_2^-$ and $Si_2OC_5H_{15}^+$ (m/z = 43, 59, 60, 73, 75 and 147). (Dietrich, R., Grobe, J., Meyer, K., Hagenhoff, B., & Benninghoven, A. Fresenius' Journal of Analytical Chemistry 1994, 349(1-3), 221-222; Gardella, J. a., & Hercules, D. M. , Fresenius' Zeitschrift Für Analytische Chemie 1981, 308(3), 297-303; Karen, A., Man, N., Shibamori, T., & Takahashi, K., Applied Surface Science 2003, 203-204, 541-546). The PFM modified samples exhibit the typical methacrylate low mass fragments $-C_2H_3^+$ and $C_2H_5^+$ (m/z = 27 and 29), **Figure 1A**. Due to interference of PDMS signals and PFM signals on the positive spectra; in this type of samples (PDMS based) the negative spectra can be more interesting to elucidate the fluorinated ring in the negative ion spectra, **Figure 1B.** Negative ion spectra shows the fluorinated fragments related with the fluorinated ring -F- and $F_2H^-$ (m/z = 19 and 49).

It can be observed an overlapping of the characteristic glucosamine peak (m/z = 72 and 180) (Kerwin, J. L., Whitney, D. L., & Sheikh, A. Insect Biochemistry and Molecular Biology 1999, 29(7), 599-607; Pastorini, E., et al., Analytica Chimica Acta 2011, 695(1-2), 77-83) with the PDMS mass fragments when Glucosamine sample (PDMS coated with PFM and incubated with glucosamine) is analyzed. Finally, silver samples present more simple spectra with the characteristic silver peaks $-Ag^+$ (m/z = 107 and 109).

EXAMPLE 2

*Silver release*

As is well known, the antibacterial activity of silver is not attributed to metallic element but to the silver ion ($Ag^+$). Its activity mechanism is in currently controversy however; it seems to be related with the complexation ability between silver ion and cysteine, interaction with DNA, cell disruption or inhabitation of electron transport. At this point, the study of the silver ion release is performed by incubation the silver coated samples in TSB and milliQ water.

The silver release of silicon wafers and PDMS samples are illustrated in **Figure 2.** Initially, there is a maximum release of $Ag^+$ about 0.6 and 1.1 ppm (from TSB and milliQ media respectively) between the first 24 h of immersion. During the following 10 days of immersion, a concentration decay and a subsequent stabilization of silver ion release about 0.1 ppm can be observed. This initial burst of silver is very interesting due to its role as extremely antibacterial concentration when implantation of the medical device is performed. High concentrations of silver ions in a short period of time can be a tremendous advantage to enhance the antibacterial activity once the device is implanted on the body and destroy the possible previous infection.

EXAMPLE 3

*Bacterial cultures*

In order to test the antibacterial activity of the modified samples, PDMS samples are incubated using *S. aureus* and *P. aeruginosa* as Gram-positive and Gram-negative representatives respectively. The bacterial growth can be significantly observed on the non-modified samples even, on the PA01 strain, biofilm structures can be observed. However, on the modified samples, the antibacterial effect of the silver ions can be observed. In **Figure 3A** it can be observed that all modified samples had a decrease on bacteria viability presenting all bacteria dead. These tremendous antibacterial activity is directly related to the silver ions solubilized on the media and even to silver coat below the bacteria (Stevens, K. N. J., et al., Biomaterials 2009, 30(22), 3682-90) that can interact with the biofilm structure from below and cause bacterial dead. Additionally, no extra bacteria adhesion on the surface can be observed as a consequence of the high antibacterial action of the silver ions and due to the hydrophobic surfaces caused by the nanostructures of the silver coating. Besides, after incubation with different strains, the supernatant were recollected and incubated again in order to observed the activity of the silver ion. In **Figure 3B** can be observed the reduction in viability of both bacteria strains, about 15% and 45% for Gram negative and Gram positive respectively. These results are consistent with the silver ion antibacterial activity where the antibacterial activity is caused by the silver ion and not by the silver metal. In addition, an interesting resistance effect can be observed regarding to type of strains and shows that Gram positive was more resistive than Gram negative probably due to the barrier effect of the cellular wall.

In fact, these results show high antibacterial activity to cause complete death, or at least minimize considerably bacterial colonization and biofilm formation. As a result, these functional surfaces can be excellent candidates to created implantable materials with antibacterial properties.

EXAMPLE 4

*Nanoparticles*

**Figure** 4 shows the *in vitro* assay of anti-proliferative effect of paclitaxel-loaded nanoparticles with different human cell lines. It has been observed that in Human Normal Lung Fibroblast (IMR-90), free drug loaded nanoparticles shows a slight decrease in cell viability but the effect is more dramatic with paclitaxel loaded nanoparticles. The same profile is observed when Stenotic fibroblasts are treated with nanoparticles. Concluding that, in both cases, drug free nanoparticles showed no obvious cytotoxicity confirming the biocompatibility of the nanoparticle polymer. However both drug loaded nanoparticles (1% and 3%) with a total amount of drug above 14 nM, showed a decrease in cell viability (about 80% for both, 1% and 3% in case of IMR-90; and 70% and 80% for 1% and 3% respectively in case of Stenotic fibroblast) related to the release of paclitaxel in the media; (10 nM and 20 nM exhibit the same results). Moreover, during the treatment it has been observed a progressive reduction in cell viability caused by the accumulation of paclitaxel on the media thus increasing the drug concentration to total amount of 14 nM. Additionally, comparing drug loaded nanoparticle treatment with pure paclitaxel it can be observed similar behavior but it can be appreciated that drug-loaded nanoparticle improves cytotoxicity effect supporting the cell decreasing viability caused by the effect of the paclitaxel in the media. Finally, it may be concluded that in both cases, IMR-90 and Stenotic fibroblast, cell viability is highly influenced by the specific effect of paclitaxel causing a significant cell decrease.

**Claims**

**1.** A method for coating a substrate with a metallic ion comprising the steps of

(i) contacting at least a first surface of the substrate with a reactive monomer containing an activated carboxyl group under conditions adequate for the formation of a polymer coat containing said activated carboxyl group on the surface of the substrate by polymerization of the monomer,
(ii) contacting the substrate obtained in step (i) with a reducing carbohydrate comprising a group reactive with said activated carboxyl group under conditions adequate for the formation of a covalent bond between the reactive group in the carbohydrate and the activated carboxyl group on the surface of the substrate, thereby obtaining an surface modified with a reducing carbohydrate and
(iii) contacting the substrate obtained in step (ii) with a salt of said metallic ion under conditions adequate for the reduction of the metallic ions by the reducing carbohydrate and the deposition of the ions in metallic form on the surface, thereby obtaining a metal-coated surface.

**2.** The method according to claim 1 wherein the substrate is a silicone-based polymer.

**3.** The method according to claims 1 or 2 wherein the group reactive with the activated carboxyl group is an amino group and wherein the covalent bond between the amino group in the carbohydrate and the activated carboxyl group on the surface of the substrate is an amide bond.

**4.** The method according to any of claims 1 to 3 wherein the reactive monomer containing a reactive carboxyl group is an activated methacrylate.

**5.** The method according to any of claims 1 to 4 wherein the contacting in step (i) is carried out using chemical vapor deposition.

**6.** The method according to any of claims 1 to 5 wherein the metallic ion is a silver ion.

**7.** The method according to any of claims 1 to 6 wherein the reducing carbohydrate comprising a reactive group is N-glucosamine.

**8.** A method for modifying the surface of a substrate with a particle selected from a nanoparticle and a microparticle comprising the steps of

(i) contacting at least a first surface of the substrate with a reactive monomer containing an activated carboxyl group under conditions adequate for the formation of a polymer coat containing said activated carboxyl group on the surface of the substrate by polymerization of the monomer and

(ii) contacting the substrate obtained in step (i) with a particle modified with a group reactive with said activated carboxyl group, wherein said particle is selected from a nanoparticle and a microparticle, wherein said particle contains at least a therapeutically active compound, and wherein said contacting is carried out under conditions adequate for the formation of a covalent bond between the reactive group in the particle and the activated carboxyl group on the surface of the substrate, thereby resulting in the modification of the second surface with particles.

**9.** The method according to claim 8 wherein the substrate is a silicone-based polymer.

**10.** The method according to any of claims 8 or 9 wherein the reactive monomer containing a reactive carboxyl group is an activated methacrylate.

**11.** The method according to any of claims 8 to 10 wherein the contacting in step (i) is carried out using chemical vapor deposition.

**12.** The method according to any of claims 8 to 11 wherein the particle is a polyethylene glycol-polyester particle.

**13.** The method according to any of claims 8 to 12 wherein the group reactive with the activated carboxyl group is an amino group and wherein the covalent bond between the amino group in the particle and the activated carboxyl group on the surface of the substrate is an amide bond.

**14.** A substrate obtainable by a method according to any of claims 1 to 13, wherein the substrate is a stent.

**15.** The stent according to claim 14, wherein a first surface has been coated with a metallic coat and wherein a second surface has been coated with particles, and wherein the surface containing the metallic coat is the inner surface and the surface containing the particle coat is the external surface.

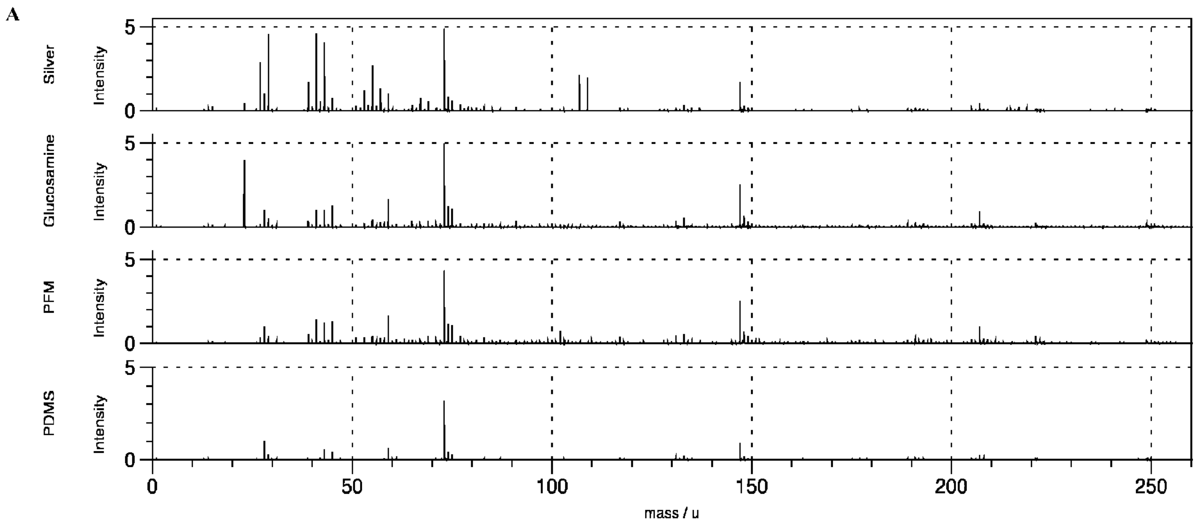
A
Silver
Glucosamine
PFM
PDMS
Intensity
5
0
0
50
100
150
200
250
mass / u

Fig. 1

B

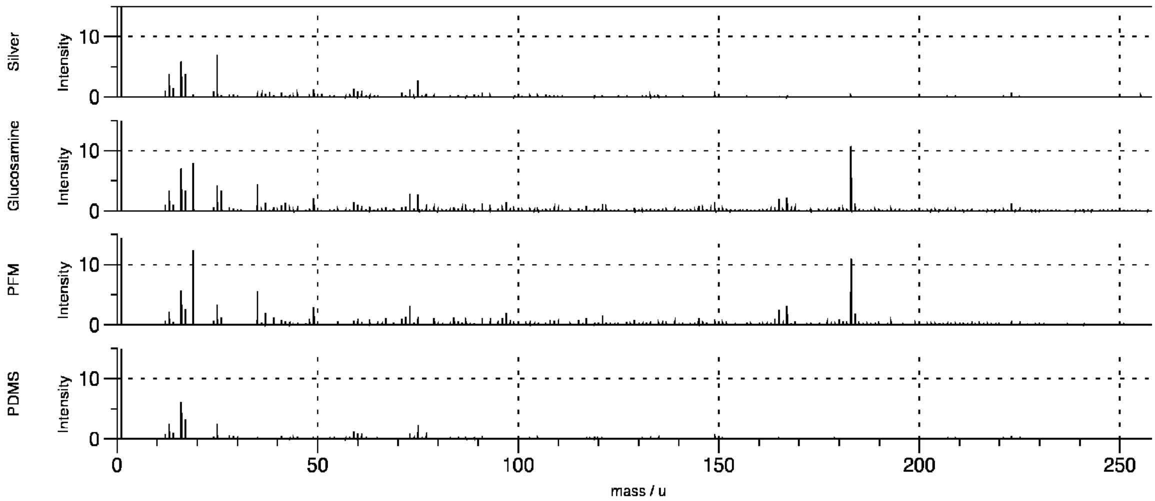
Silver
Intensity
Glucosamine
PFM
PDMS
10
0
50
100
150
200
250
mass / u

Fig. 1 (CONT.)

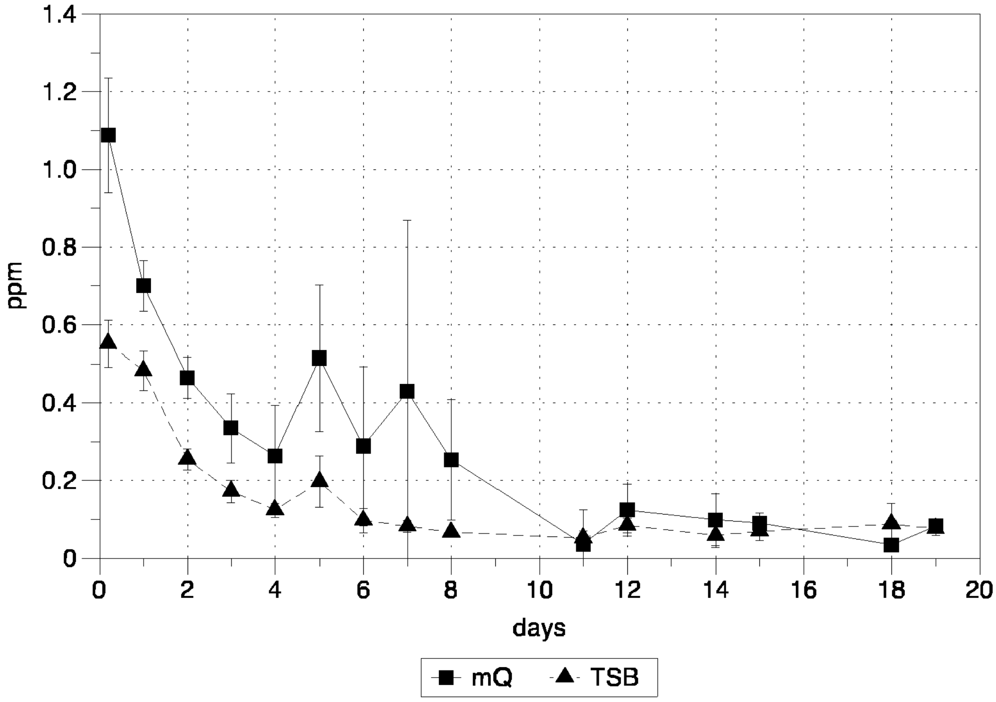
1.4
1.2
1.0
0.8
0.6
0.4
0.2
0
ppm
0
2
4
6
8
10
12
14
16
18
20
days
mQ
TSB

Fig. 2

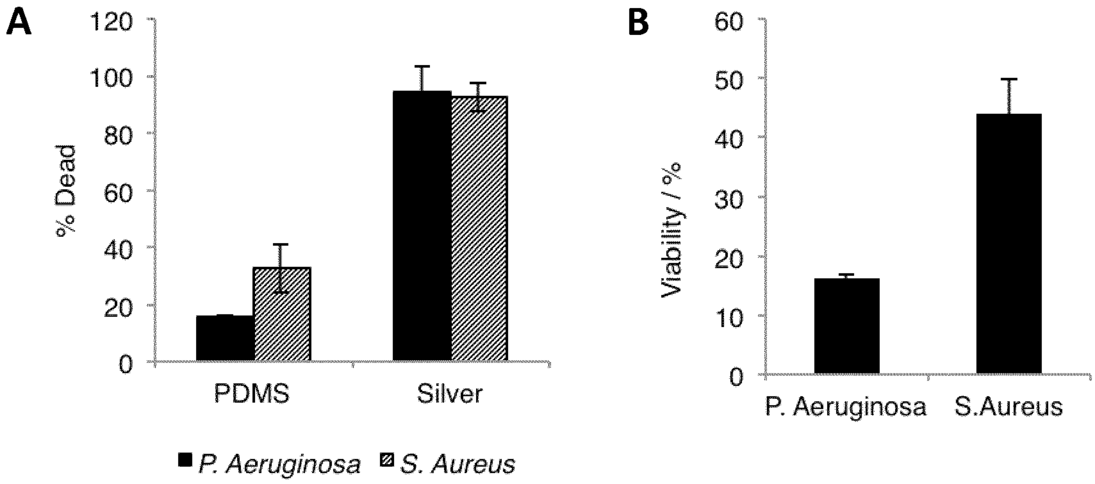
A
120
100
80
60
40
20
0
% Dead
PDMS
Silver
P. Aeruginosa
S. Aureus
B
60
50
40
30
20
10
0
Viability / %
P. Aeruginosa
S.Aureus

Fig. 3

A

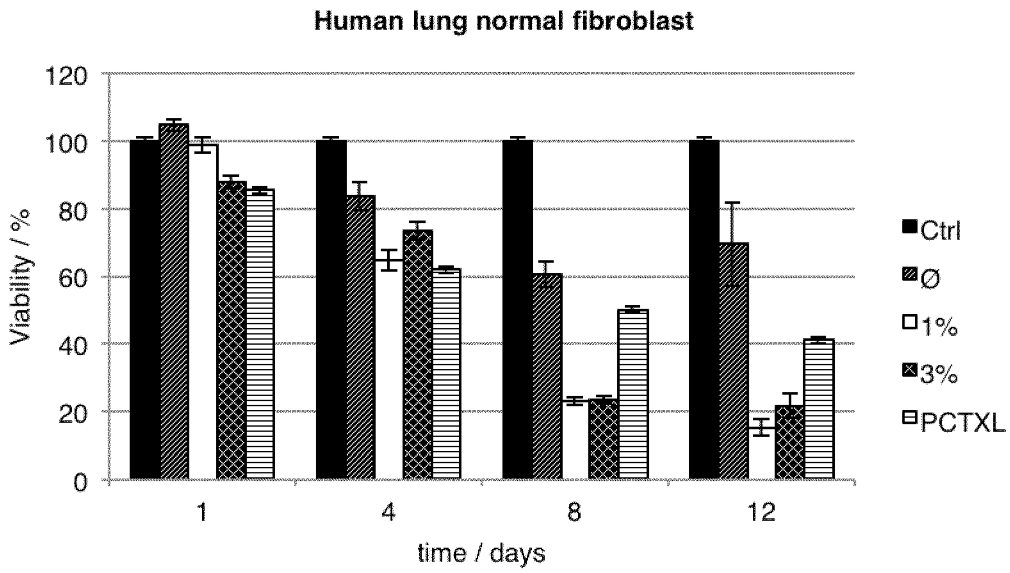
Human lung normal fibroblast
120
100
80
60
40
20
0
Viability / %
1
4
8
12
time / days
Ctrl
Ø
1%
3%
PCTXL

B

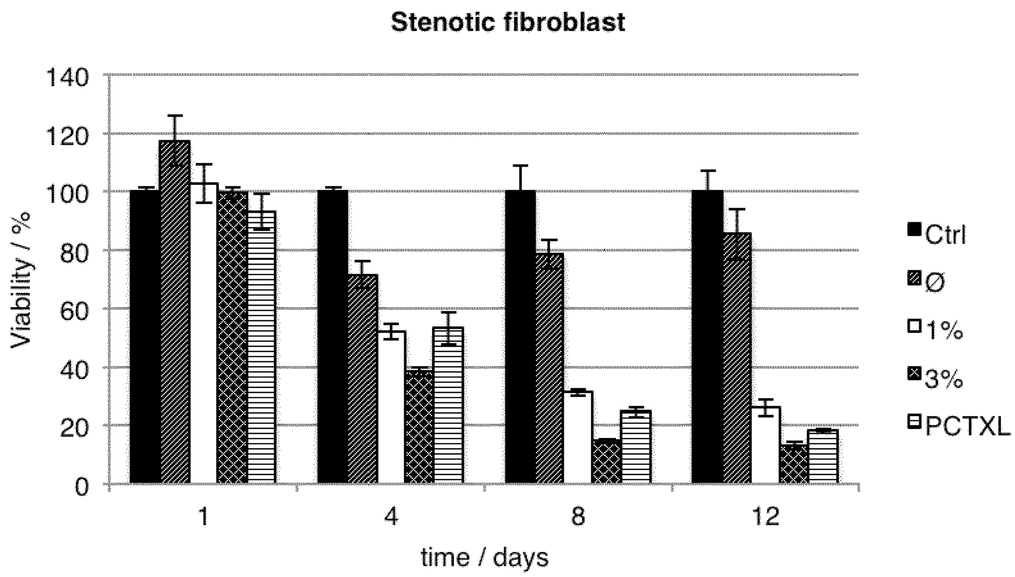
Stenotic fibroblast
140
120
100
80
60
40
20
0
Viability / %
1
4
8
12
time / days
Ctrl
Ø
1%
3%
PCTXL

**Fig. 4**

Europäisches Patentamt
European Patent Office
Office européen des brevets

# EUROPEAN SEARCH REPORT

Application Number
EP 14 38 2315

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LUIS DUQUE ET AL: "Immobilization of Biomolecules to Plasma Polymerized Pentafluorophenyl Methacrylate", BIOMACROMOLECULES, vol. 11, no. 10, 11 October 2010 (2010-10-11), pages 2818-2823, XP055163085, ISSN: 1525-7797, DOI: 10.1021/bm100910q<br>* Experimental Section ; Results and Discussion *<br>----- | 1-7,14, 15 | INV.<br>A61L31/10<br>A61L31/16<br>C23C18/16<br>C23C18/18<br>C23C18/20<br>B05D1/00 |
| A | ANNA CIFUENTES-RIUS ET AL: "Tailoring Carbon Nanotubes Surface for Gene Delivery Applications", PLASMA PROCESSES AND POLYMERS, vol. 11, no. 7, 16 July 2014 (2014-07-16), pages 704-713, XP055162637, ISSN: 1612-8850, DOI: 10.1002/ppap.201300167<br>* Sections 2.4.1. and 3.1.2. *<br>----- | 1-7,14, 15 | |
| A | US 7 201 935 B1 (CLAUDE CHARLES D [US] ET AL) 10 April 2007 (2007-04-10)<br>* column 5, line 36 - column 6, line 67 *<br>----- | 1-7,14, 15 | TECHNICAL FIELDS SEARCHED (IPC)<br>A61L<br>C23C<br>B05D |
| A | US 2014/170298 A1 (TERRY RICHARD N [US] ET AL) 19 June 2014 (2014-06-19)<br>* paragraph [0056] *<br>* claim 1 *<br>----- | 1-7,14, 15 | |

~~The present search report has been drawn up for all claims~~

2

| Place of search | Date of completion of the search | Examiner |
|---|---|---|
| The Hague | 30 January 2015 | Le Hervet, Morgan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 14 38 2315

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-7(completely); 14, 15(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number

EP 14 38 2315

The Search Division considers that the present European patent application does not comply with the requirements of unity of invention and relates to several inventions or groups of inventions, namely:

1. claims: 1-7(completely); 14, 15(partially)

   A method of coating a substrate with a metallic coating

   ---

2. claims: 8-13(completely); 14, 15(partially)

   A method for modifying the surface of a substrate with a particle

   ---

# ANNEX TO THE EUROPEAN SEARCH REPORT ON EUROPEAN PATENT APPLICATION NO. EP 14 38 2315

This annex lists the patent family members relating to the patent documents cited in the above-mentioned European search report. The members are as contained in the European Patent Office EDP file on
The European Patent Office is in no way liable for these particulars which are merely given for the purpose of information.

30-01-2015

| Patent document cited in search report | Publication date | Patent family member(s) | Publication date |
|---|---|---|---|
| US 7201935 B1 | 10-04-2007 | NONE | |
| US 2014170298 A1 | 19-06-2014 | AU 2011332884 A1 | 04-07-2013 |
| | | CA 2819030 A1 | 31-05-2012 |
| | | EP 2643099 A1 | 02-10-2013 |
| | | JP 2013544972 A | 19-12-2013 |
| | | US 2013231640 A1 | 05-09-2013 |
| | | US 2014170298 A1 | 19-06-2014 |
| | | WO 2012071536 A1 | 31-05-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03101621 A
- WO 03097245 A
- WO 2004113304 A


### Non-patent literature cited in the description


- **CHOONG CK et al.** *J Thorac Cardiovasc Surg.,* 2006, vol. 131 (1), 60-4
- **MALDONADO F et al.** *Laryngoscope,* 2014, vol. 124 (2), 498-503
- **ORTIZ R et al.** *Eur J Pediatr Surg,* 2014, vol. 24 (1), 39-45
- **ZHU GH et al.** *Laryngoscope,* 2011, vol. 121 (10), 2234-9
- **CHAO YK et al.** *Chest,* 2013, vol. 144 (1), 193-9
- **DUQUE et al.** *Plasma Prowess. Polym.,* 2010, vol. 7, 915-925
- **FRANCESCH; E. et al.** *Plasma Process. Polym.,* 2005, vol. 2, 605-611
- **LANGER ; TIRRELL.** *Nature,* 2004, vol. 428, 487-492
- **DUQUE et al.** *Biomacromolecules,* 2010, vol. 11, 2818-2823
- **DI MAURO, P. P. ; BORRÓS, S.** *Pharmaceutical Research,* 2014
- **KLAGSBRUN ; D'AMORE.** *Annu. Rev. Physiol.,* 1991, vol. 53, 217-39
- **STREIT ; DETMAR.** *Oncogene,* 2003, vol. 22, 3172-3179
- **FERRARA ; ALITALO.** *Nature Medicine,* 1999, vol. 5 (12), 1359-1364
- **TONINI et al.** *Oncogene,* 2003, vol. 22, 6549-6556
- **SATO.** *Int. J Clin. Oncol.,* 2003, vol. 8, 200-206
- **MA P. et al.** *J. Nanomed. Nanotechol.,* 2013, 4-2
- **DIETRICH, R. ; GROBE, J. ; MEYER, K. ; HAGENHOFF, B. ; BENNINGHOVEN, A.** *Fresenius' Journal of Analytical Chemistry,* 1994, vol. 349 (1-3), 221-222
- **GARDELLA, J. A. ; HERCULES, D. M.** *Fresenius' Zeitschrift Für Analytische Chemie,* 1981, vol. 308 (3), 297-303
- **KAREN, A. ; MAN, N. ; SHIBAMORI, T. ; TAKAHASHI, K.** *Applied Surface Science,* 2003, vol. 203-204, 541-546
- **KERWIN, J. L. ; WHITNEY, D. L. ; SHEIKH, A.** *Insect Biochemistry and Molecular Biology,* 1999, vol. 29 (7), 599-607
- **PASTORINI, E. et al.** *Analytica Chimica Acta,* 2011, vol. 695 (1-2), 77-83
- **STEVENS, K. N. J. et al.** *Biomaterials,* 2009, vol. 30 (22), 3682-90